Europäisches Patentamt

(19) **European Patent Office**

**Office européen des brevets**

(11) Numéro de publication: **0 161 137**
**B1**

(12) # FASCICULE DE BREVET EUROPEEN

(45) Date de publication du fascicule du brevet:
**01.06.88**

(51) Int. Cl.⁴: **A 61 M 16/00**

(21) Numéro de dépôt: **85400584.0**

(22) Date de dépôt: **26.03.85**

(54) **Appareil producteur d'air chaud pour inhalations.**

(30) Priorité: **27.03.84 FR 8404742**

(43) Date de publication de la demande:
**13.11.85 Bulletin 85/46**

(45) Mention de la délivrance du brevet:
**01.06.88 Bulletin 88/22**

(84) Etats contractants désignés:
**AT BE CH DE FR GB IT LI LU NL SE**

(56) Documents cités:
**DE - A - 2 810 325**
**DE - C - 634 919**
**FR - A - 1 022 042**
**US - A - 1 771 366**
**US - A - 2 091 034**
**US - A - 3 894 537**
**US - A - 4 412 537**

(73) Titulaire: **Seilinger, Alexandre, 2 Square de la Baume Rocquencourt, F-78150 Le Chesnay (FR)**

(72) Inventeur: **Seilinger, Alexandre, 2 Square de la Baume Rocquencourt, F-78150 Le Chesnay (FR)**

(74) Mandataire: **Dupuy, René Gaston, Cabinet René G. Dupuy & Jean M.L. Loyer 14, Rue La Fayette, F-75009 Paris (FR)**

ACTORUM AG

# Description

L'invention se rapporte à un appareil producteur d'air chaud pour inhalation.

Il est bien connu que l'air chauffé à température suffisante permet le traitement des rhinites virales, dont les agents se développent mal au-dessus de 36°, et des rhinites allergiques, selon un processus imparfaitement élucidé.

Il existe sur le marché un certain nombre d'appareils du type inhalateur comportant un ventilateur soufflant de l'air sur une résistance électrique, la totalité du flux d'air chaud étant directement dirigé vers un masque bucco-nasal (par exemple FR-A-1 083 197), ces appareils étant plus ou moins présentés comme médicaux (FR-A-1 555 598). Il a été constaté que ces appareils ne répondaient pas aux besoins respiratoires des patients, le volume d'air chaud produit étant trop important, d'où il résulte une sorte de suffocation et dont l'utilisateur ne peut se débarrasser qu'en écartant le masque avec conséquence une entrée d'air froid. Il a donc été proposé des appareils permettant d'éliminer une partie de cet air chaud.

Selon le DE-C-634 919 le flux de l'air froid vers le patient peut être régler à l'aide des clapets.

L'US-A-2 091 034 prévoit de placer au voisinage de la partie buccale d'inhalation des dérivations de sorties que le brevet prétend être destinées à éliminer le surplus de l'air non-aspiré. Manifestement c'est le flux entier qui atteint le patient et ce surplus ne peut être éliminé que par refoulement dû à l'expiration de celui-ci. Le CH-A-381 362 propose lui de pourvoir la chambre recevant le flux d'air chaud d'une part d'une soupape et d'autre part d'une mise à l'air libre, la soupape agissant comme un régulateur de pression et la mise à l'air libre comme un régulateur à la fois de pression et de température.

Le problème se complique lorsque les praticiens eurent remarqué qu'il ne suffit pas de fournir au malade de l'air chaud, mais qu'il fallait que celui-ci soit à pression et température stabilisées quel que soit le volume consommé.

A la suite de cette constatation les constructeurs ont imaginé des appareils dans lesquels on agissait sur la production d'air chaud au moyen de régulateurs fort compliqués et fort chers, sans atteindre souvent le but recherché.

Il apparait en effet qu'une pression stabilisée ne pourra être effectivement atteinte si les flux d'inspiration et d'expiration et rencontrent.

Cela explique pourquoi les appareils décrits par les brevets US-A-2 091 034 et CH-A-381 362 doivent être considérés comme inefficaces – il leur manque des moyens permettant de fractionner le volme d'air afin d'obtenir un flux à inhaler à pression stabilisée.

La présente invention concerne un appareil générateur d'air chaud, à usage médical, comportant un dispositif permettant d'obtenir d'une manière très simple et très économique de l'air chaud pulsé à température et pression stabilisées.

Le principe de l'appareil est de produire en grande quantité de l'air chaud et de n'en utiliser qu'une très faible quantité pour les besoins du traitement.

Pour atteindre ce résultat d'une manière économique dans un appareil qui comprend trois éléments essentiels:

– un générateur d'air chaud comportant une source d'air sous pression constante (ventilateur pulsant par exemple) et une résistance électrique en contact avec cet air sous pression,

– une chambre intermédiaire recevant la totalité de l'air chaud et ouverte à l'atmosphère d'une manière réglable,

– un dispositif d'inhalation, on dispose entre la chambre intermédiaire et le dispositif d'inhalation, un moyen limitant le débit d'entrée de l'air chaud dans cette dernière chambre à une valeur infime par rapport au flux d'air chaud évacué dans l'atmosphère.

D'autres particularités et avantages apparaitront à la lecture de la description et des revendications qui suivent faites en regard des dessins annexés sur lesquels:

La fig. 1 est une vue en élévation d'un mode de réalisation de l'appareil côté opposé au visage du patient;

la fig. 2 est une coupe partielle suivant 2–2 de la fig. 1;

la fig. 3 est une vue partielle en élévation du côté du visage du patient.

Selon le mode de réalisation donné à titre d'exemple non limitatif, visible sur ces figures, l'appareil 1 producteur d'air chaud, conforme à l'invention comprend d'une part dans un boîtier 1, un générateur d'air chaud 2 formé d'une source d'air sous pression, par exemple un ventilateur électrique, et d'une résistance électrique (non représentés) et d'autre part un corps tubulaire 3 et un dispositif d'inhalation 4 – représenté par un masque nasal. Bien entendu le ventilateur pourrait être remplacé par un autre type de source d'un air sous pression, par exemple un circuit d'air du genre de ceux utilisé dans les centres hospitaliers. De même le masque nasal pourrait être remplacé par des injecteurs à enfoncer dans le nez, ou à placer sous le nez du patient.

Le corps tubulaire 3 communiquant d'une part avec le générateur 2 et d'autre part avec le dispositif d'inhalation 4 est aménagé en chambre intermédiaire recevant la totalité du flux d'air chaud produit.

Cette chambre intermédiaire C est mise à l'atmosphère au moyen d'un dispositif réglable. A cet effet la paroi du corps tubulaire 3 est percée d'un orifice 3a pouvant être plus ou moins obturé par un volet mobile constituée par une bague 7 percée d'au moins un orifice 7a (ou d'une pluralité d'orifices de sections différentes).

Le réglage s'effectue par rotation de la bague 7 de manière à faire coïncider plus au moins les orifices 3a et 7a. Un ergot 9 fixé sur le corps 3 et parcourant une boutonnière 7b de la bague 7 limite le déplacement de la bague par rapport au corps creux, pénètre dans une boutonnière 10 de la bague. Lorsque, comme indiqué sur les trois figures, l'ergot 9 est en butée sur une extrémité de la boutonnière 7b les axes des orifices 3a et 7a coïncident et la section d'évacuation de l'air chaud est alors maximale. Si on fait tourner la bague 7 on obture partiellement l'orifice 3a et lorsqu'on amène l'autre extrémité de la boutonnière 7b en butée sur l'ergot 9 la section d'évacuation de l'air chaud est minimale.

La chambre intermédiaire C formée à l'intérieur du corps tubulaire 3 communique par un orifice avec le dispositif d'inhalation 4 au travers d'un organe régulateur de débit essentiel à l'invention.

Comme précédemment dit, le principe de l'appareil constitue à ne prélever sur le flux total qu'une infime partie de celui-ci pour l'inhalation. Par ailleurs cet organe régulateur de débit, dont la perte de charge est connue permet d'obtenir dans le dispositif inhalateur, ici le masque nasal 4 une pression résiduelle et une température pratiquement stabilisées.

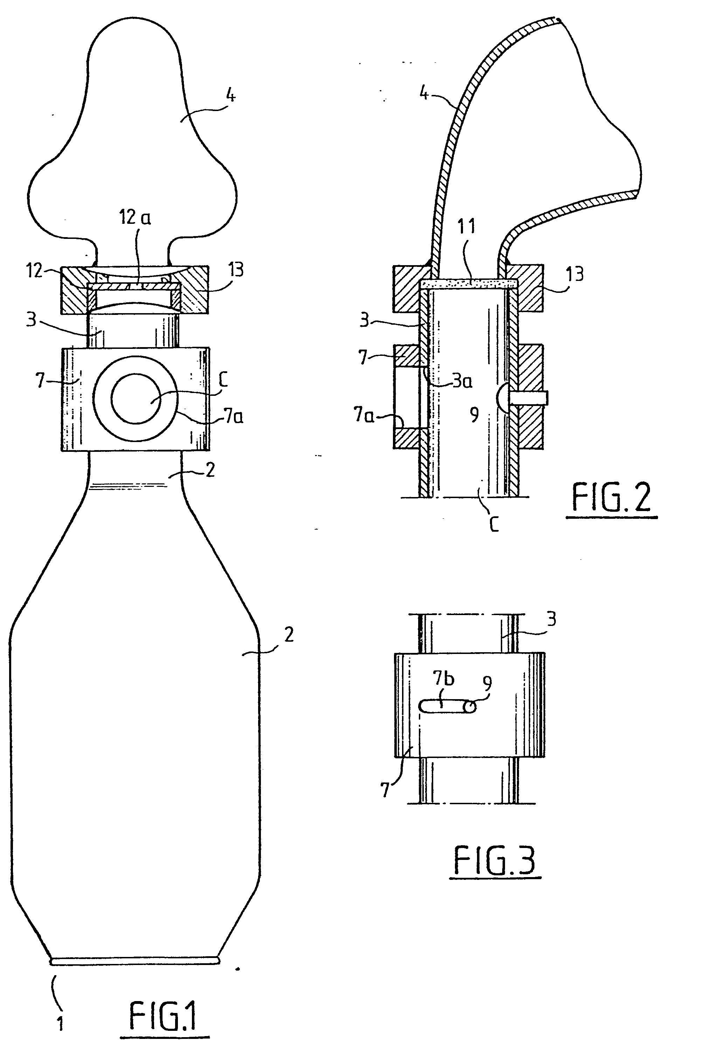

Comme on le voit sur la fig. 1 la communication entre la chambre C et le masque se fait par un diaphragme 12 constitué par un opercule percé d'un orifice calibré 12a placé au travers de l'orifice de prélèvement.

Ce diaphragme à orifice calibré peut être remplacé par des dispositifs jouant le même rôle régulateur de débit.

Ces dispositifs peuvent être soit un filtre 11 (fig. 2) soit une cartouche contenant des produits médicamenteux pouvant s'évaporer ou se sublimer.

En donnant à ce filtre (ou à cette cartouche) une section utile de passage d'air appropriée, il (ou elle) peut constituer l'orifice de petite section utile permettant le prélèvement d'air dans la chambre intermédiaire.

Il est aisé de comprendre que la section utile de passage de l'air au travers de l'orifice calibré 12a ou au travers d'un filtre ou d'une cartouche 11 à perte de charge contrôlée est très petite comparée à la section de l'orifice 3a d'évacuation du flux principal d'air chaud, même quand cet orifice est partiellement obturé par la bague 7. Le diaphragme 12, ou la cartouche 11 est maintenu en plan par une bague 13 sur laquelle est soudé un masque 4 destiné à recouvrir le nez du patient, la bouche n'étant pas recouverte.

Les caractéristiques du générateur 2 étant constantes et l'orifice 3a étant grand ouvert, on obtient dans la chambre C une température et une pression constantes quelle que soit la quantité d'air consommée par le patient. Si on fait tourner la bague 7 pour obturer partiellement l'orifice 3a, on réduit le débit d'air de sortie du flux principal ce qui a pour conséquence d'augmenter la température et la pression dans la chambre C; à chaque position de la bague 7 ne correspondent qu'une seule température et qu'une seule pression indépendantes de la consommation d'air.

On peut apporter certaines modifications à l'appareil en fonction des résultats spéciaux désirés.

Par exemple il peut être intéressant de filtrer l'air sans que le filtre constitue l'orifice de petite section utile permettant le prélèvement d'air dans la chambre intermédiaire. Dans ce cas on peut le disposer soit au niveau de l'orifice de prélèvement de l'air extérieur soit entre le générateur d'air chaud et la chambre intermédiaire.

L'appareil permet également l'inhalation d'air humide. Il suffit alors soit de remplacer la cartouche décrite ci-dessus par un évaporateur d'au alimenté ou non par un petit réservoir annexe soit de faire pénétrer dans le dispositif d'inhalation par un orifice approprié de la vapeur d'eau produite par un générateur extérieur.

Dans le cas du traitement à l'air sec de la rhinite il peut être intéressant de disposer d'un léger réglage de la température pour tenir compte du patient et de son état.

Par ailleurs, le générateur produisant une élévation de température sensiblement constante de l'air ambiant il peut être nécessaire de modifier cette élévation de température si l'air ambient n'est pas à la température normale de 19/20°.

Enfin si plusieurs types d'inhalateur ou de filtres ou de cartouches peuvent être utilisés avec le même ensemble constitué par le générateur d'air chaud et par la chambre intermédiaire C, il peut être nécessaire de disposer d'une gamme de température de l'air dans ladite chambre intermédiaire en particulier pour tenir compte des calories absorbées soit par l'évaporation d'eau soit par l'évaporation ou la sublimation de substances médicamenteuses.

Tous ces problèmes sot résolus grâce aux possibilités de réglage de la sortie 3 en agissant sur la bague 7, une bague disposée autour de la chambre intermédiaire permet de régler le débit de l'air qui s'échappe par le grand orifice en l'obsturant plus ou moins et ainsi d'obtenir dans la chambre intermédiaire C la température d'air nécessaire pour que l'air inhalé soit à la température voulue.

On doit remarquer que les moyens de réglage 11–12 permettent d'obtenir à l'intérieur du dispositif d'inhalation une pression légèrement supérieure à la pression expiratoire du patient.

**Revendications**

1. Appareil producteur d'air chaud pour inhalation à pression et température stabilisées, comportant une source (2) d'air sous pression constante, une résistance électrique en contact avec cet air sous pression, une chambre intermédiaire (6) recevant la totalité de l'air chaud et ouverte à l'atmosphère par un orifice (3a, 7a) et un dispositif d'inhalation (4) qui communique avec la chambre intermédiaire, caractérisé en ce que l'orifice (3a, 7a) d'ouverture de la chambre intermédiaire vers l'atmosphère est réglable et ladite chambre intermédiaire communique avec le dispositif d'inhalation (4) au travers de moyens (11–12) limitant le débit l'entrée de l'air chaud dans ce dispositif à une valeur infime par rapport au flux rejeté à l'atmosphère par l'orifice d'ouverture (3a, 7a).

2. Appareil selon la revendication 1, caractérisé en ce que le dispositif d'inhalation est un masque (4) uniquement nasal.

3. Appareil selon la revendication 1, caractérisé en ce que ces moyens limitent le débit de l'air chaud dans le dispositif d'inhalation de manière à ce que cet air soit à une pression légèrement supérieure à la pression expiratoire.

4. Appareil selon l'une des revendications antérieures, caractérisé en ce que ces moyens limitant le débit résident en un diaphragme (12) percé d'un orifice de faible section (12a) par rapport à celle de l'orifice (3a) de mise à l'atmosphère.

5. Appareil selon l'une des revendications antérieures, caractérisé en ce que ces moyens limitant le débit résident en une cartouche de perméabilité contrôlée.

6. Appareil d'air chaud selon la revendication 5, caractérisé en ce que la cartouche est un filtre.

7. Appareil selon l'une quelconque des revendications précédentes, caractérisé en ce qu'il est aménagé pour fournir au dispositif d'inhalation des mélanges à l'air chaud et sec provenant de la chambre intermédiaire soit un produit solide sublimé soit un produit liquide pulvérisé ou vaporisé soit un produit gazeux.

## Patentansprüche

1. Vorrichtung zum Erzeugen von Warmluft für die Inhalierung bei konstantem Druck und konstanter Temperatur, mit einer unter konstantem Druck stehenden Luftquelle (2), mit einem mit der Druckluft in Kontakt stehenden elektrischen Widerstand, mit einer Zwischenkammer (C), die die gesamte erwärmte Luft aufnimmt und zur Umgebung über eine Öffnung (3a, 7a) offen ist, und mit einer Inhaliereinrichtung (4), die mit der Zwischenkammer Verbindung hat, dadurch gekennzeichnet, dass die die Zwischenkammer mit der Umgebung verbindende Öffnung (3a, 7a) regelbar ist und die Zwischenkammer mit der Inhaliereinrichtung (4) quer durch Mittel (11–12) Verbindung hat, die die Eintrittsmenge an warmer Luft in diese Einrichtung auf einen Wert begrenzen, der sehr klein ist im Vergleich zu dem über die Öffnung (3a, 7a) in die Umgebung abgebenden Strom.

2. Vorrichtung nach Anspruch 1, dadurch gekennzeichnet, dass die Inhaliereinrichtung ausschliesslich eine nasale Maske (4) ist.

3. Vorrichtung nach Anspruch 1, dadurch gekennzeichnet, dass diese Mittel die Eintrittsmenge an erwärmter Luft in die Inhaliereinrichtung derart begrenzen, dass diese Luft den Druck aufweist, der geringfügig über dem Atmungsdruck liegt.

4. Vorrichtung nach einem der vorhergehenden Ansprüche, dadurch gekennzeichnet, dass die die Eingangsmenge begrenzenden Mittel in einem Diaphragma (12) angeordnet sind, das von einer Öffnung (12a) mit geringer Querschnittsfläche durchsetzt ist im Vergleich zu der Querschnittsfläche der Öffnung (3a) zur Umgebung.

5. Vorrichtung nach einem der vorhergehenden Ansprüche, dadurch gekennzeichnet, dass sich die Eintrittsmenge begrenzenden Mittel in einer Kartusche mit einstellbarem Durchgang befinden.

6. Warmluftvorrichtung nach Anspruch 5, dadurch gekennzeichnet, dass die Kartusche ein Filter ist.

7. Vorrichtung nach einem der vorhergehenden Ansprüche, dadurch gekennzeichnet, dass sie zur Abgabe eines Gemisches aus warmer und trockener Luft in die Inhaliereinrichtung eingerichtet ist, die aus der Zwischenkammer entweicht, und zwar ein sublimierter Feststoff, ein pulverisierter oder verdampfter Flüssigstoff oder ein gasförmiger Stoff.

## Claims

1. Device for the production of warm air for inhalation at stabilized pressure and temperature, comprising a source (2) of air at constant pressure, an electrical resistor in contact with this pressurized air, an intermediate chamber (C) which receives all the warm air and is open to the atmosphere by way of an orifice (3a, 7a), and an inhalation device (4) which communicates with the intermediate chamber, characterized in that the orifice (3a, 7a) which opens the intermediate chamber to the atmosphere can be regulated and the said intermediate chamber communicates with the inhalation device (4) via means (11–12) limiting the flow rate of entry of warm air into this device to an extremely small amount with respect to the flow expelled to the atmosphere by way of the opening orifice (3a, 7a).

2. Device according to claim 1, characterized in that the inhalation device is a mask (4) which covers only the nose.

3. Device according to claim 1, characterized in that these means limit the flow rate of warm air into the inhalation device in such a manner that this air is at a pressure slightly greater than that of the exhaled air.

4. Device according to one of the preceding claims, characterized in that these means limiting the flow rate are composed of a diaphragm (12) pierced by an orifice of small cross-section (12a) with respect to that of the orifice (3a) which expels to the atmosphere.

5. Device according to one of the preceding claims, characterized in that these means limiting the flow rate are composed of a cartridge of controlled permeability.

6. Warm-air device according to claim 5, characterized in that the cartridge is a filter.

7. Device according to any one of the preceding claims, characterized in that there is provided, for the device for inhaling mixtures of warm and dry air originating in the intermediate chamber, a sublimed solid product or a powdered or vaporized liquid product or a gaseous product.

# FIG.1

0 161 137

FIG.1

FIG.2

FIG.3

5